# EUROPÄISCHE PATENTSCHRIFT

(11) **EP 0 836 894 B1**
(45) Veröffentlichungstag und Bekanntmachung des Hinweises auf die Patenterteilung: **07.03.2001**
(21) Anmeldenummer: 97100782.8
(22) Anmeldetag: 20.01.1997
(51) Int. Cl.: B08B 3/02

(54) **Maschine zur Reinigung und/oder Desinfektion von Bedienungsgeräten des Friseurgewerbes**
Machine for cleaning and/or disinfecting of hairdresser instruments
Machine pour le nettoyage et/ou la désinfection d'instruments de coiffure

(30) Priorität: 18.10.1996 DE 19643174
(43) Veröffentlichungstag der Anmeldung: 22.04.1998
(73) Patentinhaber: Dittscheid, Bodo, sen., 66121 Saarbrücken (DE)
(72) Erfinder: Dittscheid, Bodo, sen., 66121 Saarbrücken (DE)
(74) Vertreter: Wieske, Thilo

(56) Entgegenhaltungen:
- WO-A-92/11099
- DE-A- 2 749 448
- GB-A- 2 046 086
- US-A- 3 982 296
- US-A- 4 370 992
- US-A- 4 403 364

## Beschreibung

Die Erfindung betrifft eine Maschine zur Reinigung bzw. Reinigung und Desinfektion von Bedienungsgeräten des Friseurgewerbes nach Anspruch 1 sowie ein Verfahren zur Reinigung bzw. Reinigung und Desinfektion von Bedienungsgeräten des Friseurgewerbes nach Anspruch 4.

Bei der derzeitigen Handhabung von Desinfektions- und Reinigungsmaßnahmen von Bedienungsgeräten des Friseurgewerbes ist es dem Friseur selbst überlassen, die Bediengeräte wie beispielsweise Frisierkamm, Bürste, Schere, Rasier- und Nackenpinsel etc. zu reinigen und zu desinfizieren. Es ist dabei für den Kunden nicht erkennbar, ob bei den bei ihm verwendeten Geräten eine Reinigung/Desinfektion durchgeführt wurde oder nicht.

Aus der US-PS 4,403,364 ist eine Vorrichtung zur Reinigung von Haarbürsten bekannt, die aus mehreren Kammern besteht, in denen die Bedienungsgeräte zur Reinigung und ggf. auch zur Desinfektion abgespritzt und getrocknet werden. Die Vorrichtung weist eine Abspritzvorrichtung und eine Trocknungsvorrichtung auf.

Mit der nachfolgend beschriebenen Maschine sowie dem Verfahren, dem die Funktionsweise der Maschine zugrunde liegt, soll die Reinigung bzw. Reinigung und Desinfektion erleichtert und verbessert werden.

Bei der Maschine nach Anspruch 1 sind die für einen Frisiervorgang benötigten Bediengeräte in einen oder mehrere Körbe einbringbar und der Korb bzw die Körbe ist/sind in die Maschine einbringbar zur Reinigung und gegebenenfalls Desinfektion der in diesem Korb/diesen Körben befindlichen Geräte, wobei die Maschine weiterhin eine Verschweißvorrichtung aufweist, mit der der Korb/die Körbe in einem sich an die Trocknung anschliessenden Arbeitsgang durch die Maschine durch Verschweißen mittels einer Folie verschliessbar ist/sind.

Dadurch können die Bediengeräte zu Sets zusammengefaßt werden, die gemeinsam gereinigt werden können. Durch die Verwendung der Körbe können diese Sets einfach gemeinsam eingebracht werden. Durch die Öffnungen der Körbe können die Bediengeräte in den Körben gereinigt werden.

Weiterhin ist in einfacher Weise realisiert, daß die zu den Sets zusammengefaßten Bediengeräte für den Benutzer erkennbar gereinigt worden sind, bevor dieses Set bei ihm zur Anwendung kommt. Der Benutzer kann dies daran erkennen, daß ein neues Set geöffnet wird, weil jedes in einem Korb befindliche Set nach der Trocknung verschlossen wird. Das Verschließen der Körbe erfolgt derart, dass eine Plastikfolie über den Korb geschweißt wird. Es ist dabei denkbar, daß zur Restabtrocknung einzelne Löcher des Korbes nicht von der Plastikfolie überdeckt sind, wobei durch diese Löcher keines des Bediengeräte entnehmbar sein sollte.

Dadurch entfällt die Notwendigkeit, daß der Friseur die Bediengeräte während der Reinigung/Desinfektion in der Hand halten muß. Durch die damit verbundene Vereinfachung der Reinigung/Desinfektion wird die Akzeptanz erhöht. Insbesondere kann der Friseur mit einem Set von Bediengeräten weiterarbeiten, während ein anderes Set gerade in der Maschine gereinigt wird.

Durch die verbesserte und konsequentere Reinigung kann die mögliche Übertragung von Krankheiten bzw. Krankheitserregern eingeschränkt bzw. ganz unterbunden werden.

Die Maschine nach Anspruch 2 weist mehrere Kammern auf Das Abspritzen und das Trocknen findet in verschiedenen Kammern statt.

Dadurch ist die Effizienz des Trocknungsvorganges verbessert, wenn die Trocknung in einer separaten Kammer stattfindet, in die die Bediengeräte eingebracht werden. Es kann dann nämlich der Energieaufwand vermieden werden, der nötig wäre, um die Kammer selbst zu trocknen.

Die Maschine nach Anspruch 3 weist mehrere Kammern auf, wobei in der ersten als Spritzkammer ausgebildeten Kammer die Geräte vorgereinigt werden, wobei in der zweiten als Spritzkammer ausgebildeten Kammer der Hauptspül- und Hauptwaschvorgang erfolgt unter Zugabe von Wärme und Reinigungsmittel, wobei in der dritten als Spritzkammer ausgebildeten Kammer die Bediengeräte überspült werden unter Beigabe von Desinfektionsmittel und wobei in der vierten als Trockenkammer ausgebildeten Kammer die Bediengeräte getrocknet werden unter Beimischung erwärmter Luft.

Indem Desinfektion und Reinigung in verschiedenen Kammern vorgenommen werden, kann vorteilhaft eine Mischung von Reinigungs- und Desinfektionsflüssigkeit verhindert werden.

Anspruch 4 betrifft ein Verfahren zur Reinigung bzw. Reinigung und Desinfektion von Bedienungsgeräten des Friseurgewerbes, mittels einer Maschine, die wenigstens eine Kammer aufweist, wobei die Bedienungsgeräte zur Reinigung und Desinfektion abgespritzt und getrocknet werden, wobei erfindungsgemäß nach dem Einbringen der für einen Frisiervorgang benötigten Bediengeräte in einen oder mehrere Körbe der Korb bzw die Körbe in die Maschine eingebracht werden zur Reinigung und gegebenenfalls Desinfektion der in diesem Korb/diesen Körben befindlichen Geräte, und wobei der Korb/die Körbe unmittelbar nach der Trocknung in der Maschine durch Verschweißen mittels einer Folie verschlossen wird/werden.

Dieses Verfahren betrifft die Arbeitsweise einer Maschine entsprechend der Ausgestaltung nach Anspruch 1. Die entsprechenden Vorteile sind im Zusammenhang mit Anspruch 1 bereits erläutert.

Bei dem Verfahren nach Anspruch 5 weist die Maschine mehrere Kammern auf, wobei das Abspritzen und das Trocknen in verschiedenen Kammern erfolgt.

Diese Ausgestaltung des Verfahrens betrifft die Arbeitsweise einer Vorrichtung entsprechend Anspruch 2. Die Vorteile dieses Verfahrens sind im Zusammenhang mit Anspruch 2 erläutert worden

Bei dem Verfahren nach Anspruch 6 weist die Maschine mehrere Kammern auf, wobei in der ersten als Spritzkammer ausgebildeten Kammer die Geräte vorgereinigt werden, wobei in der zweiten als Spritzkammer ausgebildeten Kammer der Hauptspül- und Hauptwaschvorgang durchgeführt wird unter Zugabe von Wärme und Reinigungsmittel, wobei in der dritten als Spritzkammer ausgebildeten Kammer die Bediengeräte überspült werden unter Beigabe von Desinfektionsmittel und wobei in der vierten als Trockenkammer ausgebildeten Kammer die Bediengeräte getrocknet werden unter Beimischung erwärmter Luft.

Die Ausgestaltung dieses Verfahrens betrifft die Arbeitsweise einer Vorrichtung entsprechend der Ausgestaltung nach Anspruch 3. Die Vorteile dieses Verfahrens sind im Zusammenhang mit Anspruch 3 erläutert

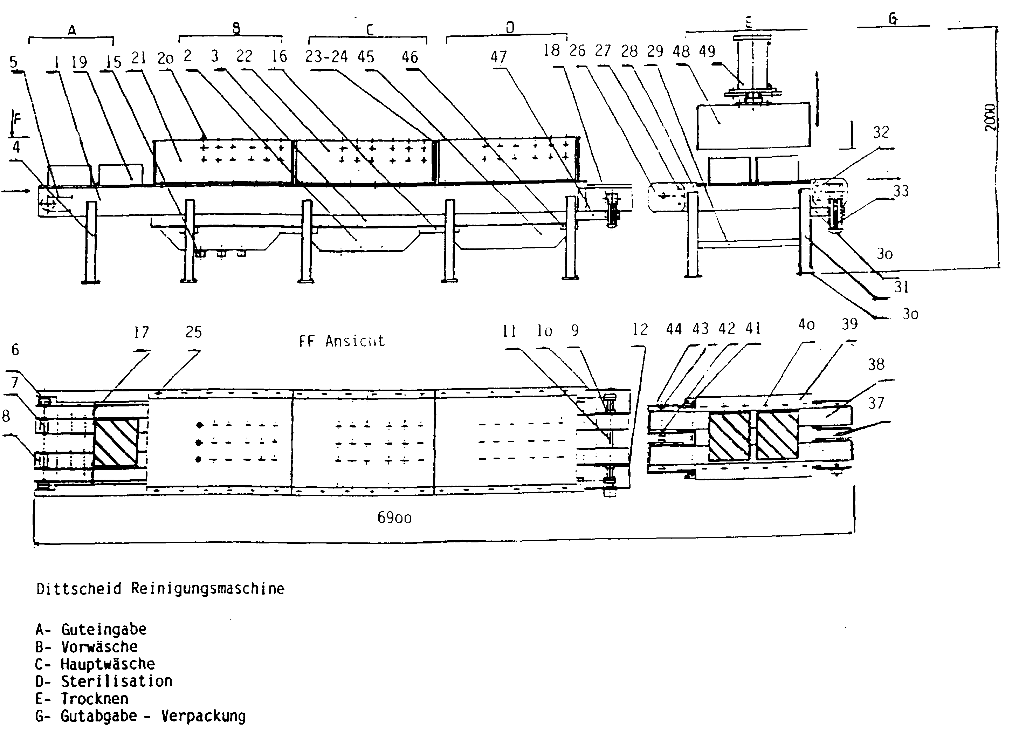

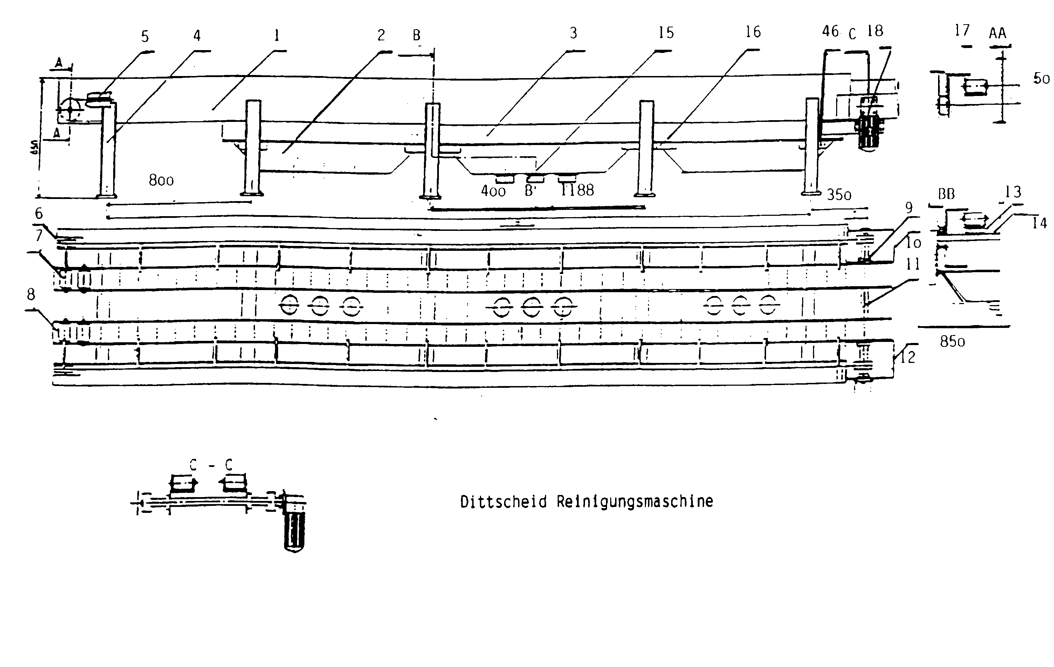

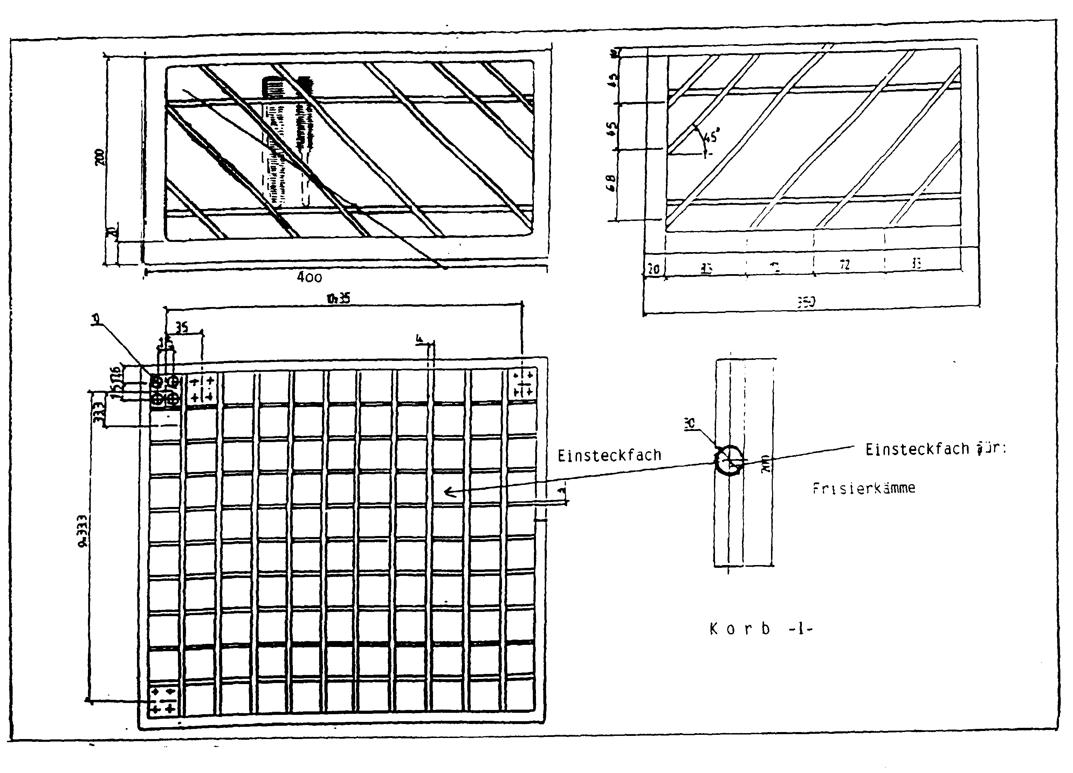

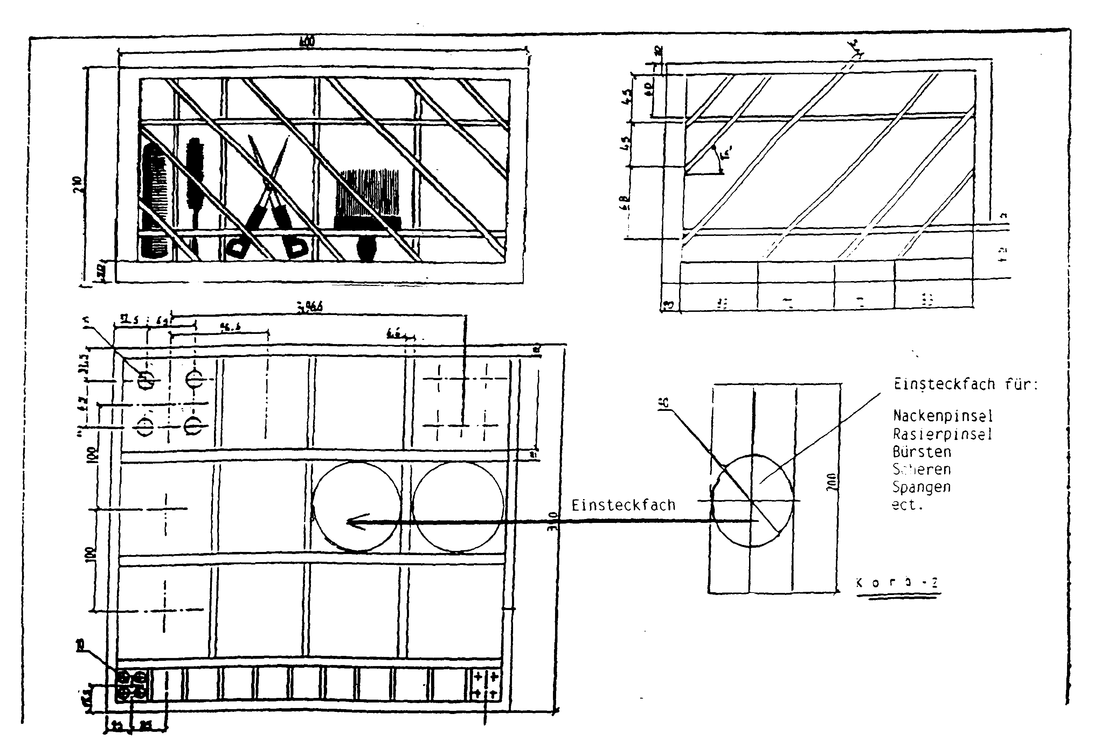

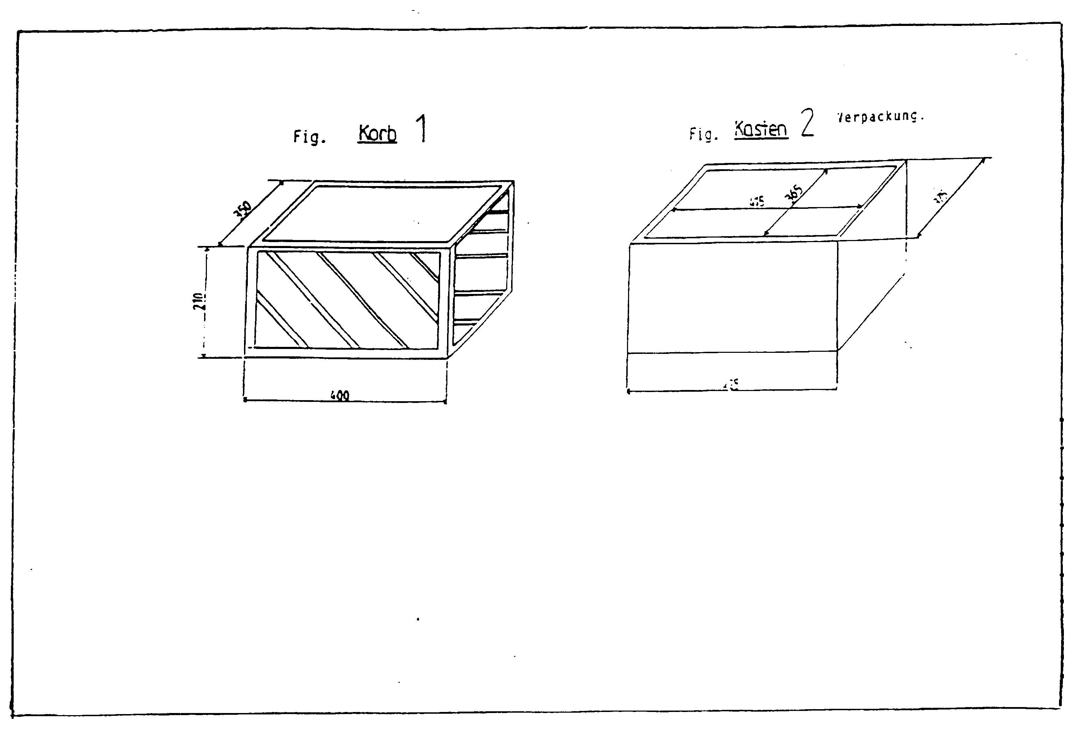

Ein Ausführungsbeispiel der Erfindung ist in der Zeichnung näher dargestellt und wird im folgenden beschrieben.

Inbetriebnahme der vollautomatisch, über Kettenzug und Relais gesteuerten Reinigungsmaschine über 4 Positionen.

### 1. Wasch- und Reinigungsvorgang.

Die mit Kämmen, Bürsten, Pinsel etc. aufgefüllten speziell gefertigten Transportkörbe (Zeichnung Korb 1 ur 2) werden auf den Rollen-Kettenzug plaziert. Zeichnung Blatt -1- Pos -5-. Nach Einschaltung der Energie und Inbetriebnahme der Reinigungsmaschine, werden die Körbe durch Mitnehmer nach Kammer -B- befördert und es beginnt nunmehr die erste gründliche Vorwäsche. 27 an den Innesenwänden der Kammer 1 installierte Wasser-Spritzdüsen Pos. -2o- überspülen gezielt und eindringend jedes in den **Einsteckfächern** der Körbe postierte Einzelteil..

Diese Grundreinigung übernimmt die Aufgabe **grobe** Staub-und Schmutzpartickel von den Frisierbestecken zu entfernen. Verbrauchtes Spülwasser wird in Auffangbecken Zeichnung Pos. -2- gesammelt, alsdann über Sieb und Verschraubung Zeichng. Pos.-15- als Abwasser in die vorhandene Kanalisation abgeleitet.

### 2. Haupt Wasch- und Reinigungsprozeß.

### Kammer -2-

Die Erfindung, einmalige Benutzung von Friseurgerätschaften, dienlich zur perfekten **Hygiene** im Frisiersalon, ordnet nunmehr die sich in Kammer -C- stattfindende Hauptreinigung an. 3o an den Innenwänden der Kammer -C- angebrachte Spritzdüsen, überspülen mit auf ca. 6o Grad Celsius erwärmtem Wasser Bestecke und die Innenwände der Transportkörbe.
Dem Wasser wird ein handelsübliches Reinigungsmittel erster Qualität dosiert zugemischt. Den Friseurgerätschaften ist höchstes Maß an Sauberkeit zugedacht.
Nach Beendigung der Hauptreinigung in Kammer -C-, schaltet ein Relais zwecks Abkühlung der Gerätschaften auf Zufuhr von Klarwasser.
Verbrauchtes Wasser wird in Abwasserbecken Pos . -2- aufgefangen und in die Kanalisation weitergeleitet.

### 3. Desinfektion PLanzeichng. Kammer -D-

Mehrmalige Verwendung von ungereinigten Friseur-Bedienungsgerätschaften birgt die Gefahr, Krankheitserreger, Bakterien vieler Art auf den Menschen zu übertragen bzw. den Organismus entscheidend negativ zu beeinflussen .

Der Kettenzug hat die Transportkörbe mit den Gerätschaften nach Kammer -D- befördert.
Auch hier übernehmen installierte Wasserspritzdüsen die Funktion, alle in den Körben eingebrachten Teile mit Desinfektionsmittel abzusprühen.
Das System, die Form der exakten Aufteilung aller Einsteckfächer garantiert die Intensität der Befeuchtung sämtlicher Bestecke und der Korbinnenwände.
Nach ca. 2 Minuten ist der Sachverhalt, die Desinfektion von Friseurgeräten beendet, die Laufkette der Dittscheid Reinigungsmaschine automatisch in Funktion gesetzt und das gesamte Material nach Planzeichnung Position -E- befördert.

### 4. Klimagesteuerte Trockenkammer. Pos. -E-

Das Gehäuse wird mittels Zylinder Position -49- angehoben und die Transportkörbe an frei gewordene Stelle plaziert.
Gleichmäßige, schonende und schnelle Trocknung an jedem Punkt der Frisierbestecke und den Transportkörben innerhalb der Trockenkammer, dabei werden die Vorteile aller fallweise rentablen Wärmerückgewinnungssysteme genutzt. Direkte Wärmerückgewinnung (Ofenabwärme, Abluft Gegenstromprinzip) ebenso wie indirekte Wärmerückgewinnung (Wärmetauscher)
Effiziente Luftführung durch gezielte Düsentrocknung.

### 5. Verpackung - Verschluß der Körbe.

### Ende des Kettenzuges, Packtisch.

Nach Beendigung des Trockenverfahrens, werden die Besteckkörbe jeweils einzeln mit Folie an obiger Korböffnung verschweißt. Während des gesamten Reinigungs- u. Desinfektionsverfahrens gab es keine persl. körperliche Berührung mit den Frisiergerätschaften. Körperkontakt erst nach Aufbrechen der Folie zum einmaligen Gebrauch im Frisiersalon.
Gereinigte und desinfizierte Friseurgerätschaften in kundenfreundlicher Verpackung zur einmaligen persönlichen Verwendung am Kunden bedeutet die höchste Stufe an Persönlichkeit und Reinheit im Frisiersalon.

**STÜCKLISTE :**

| Fig. | Meng. | Einh. | Bennenung | Norm | Blatt- | Bemrk. |
|---|---|---|---|---|---|---|
| 1 | 2 | STk. | Kettenträger | | 1.2. | Metall |
| 2 | 1 | " | Abwasserbecken | | 1.2. | Blech |
| 3 | 2 | " | Schürze | | 1.2. | Kunstst. |
| 4 | 1o | " | Gerüst | | 1.2. | Stahl |
| 5 | 2 | " | Kette | | 1.2. | Metall |
| 6 | 4 | " | Kettenrad | | 1.2. | " |
| 7 | 44 | " | Rollen | | 1.2. | " |
| 8 | 2 | " | Rollenträger | | 1.2. | " |
| 9 | 2 | " | Lager | | 1.2. | " |
| lo | 1 | " | Stütze | | 1.2. | " |
| 11 | 1 | " | Antriebswelle | | 1.2. | " |
| 12 | 1 | " | Stütze | | 1.2. | " |
| 13 | 5 | " | Stütze | | 2 | " |
| 14 | 5 | " | Träger | | 2 | " |
| 15 | 9 | " | Verschlauchung | | 1.2. | " |
| 16 | 4 | " | Stütze | | 1.2. | " |
| 17 | 23 | " | Mitnehmer | | 1.2. | " |
| 18 | 1 | " | Kettenförderer-Motor | | 1.2. | " |
| 19 | 2 | " | Korb | | 1 | Kunstst. |
| 2o | 126 | " | Düsen | | 1 | Metall |
| 21 | 4 | " | Kammerwand | | 1 | " |
| 22 | 3 | " | Düsengehäuse | | 1 | " |
| 23 | 4 | " | KLappenrahmen | | 1 | " |
| 24 | 4 | " | Klappen | | 1. | Kunstst. |
| 25 | 36 | " | Senkschrauben | DIN 963 M 1ox5o | 1 | Metell |
| 26 | 2 | " | Einfassung | | 1 | Blech |
| 27 | 16 | " | Zylinderschrauben | DIN 912-M 1ox5o | 1 | Metall |
| 28 | 2 | " | Riementräger | | 1 | " |
| 29 | 2 | " | Stab | | 1 | " |
| 3o | 1 | " | Motorstütze | | 1 | " |
| 31 | 4 | " | Gerüst | | 1 | " |
| 32 | 2 | " | Einfassung | | 1 | Blech |
| 33 | 1 | " | Antriebsmotor | | 1 | Metall |
| 34 | 2 | " | Riemenstütze A A | | 2 | Blech |
| 35 | 2 | " | Träger A A | | 2 | Metall |
| 36 | 8 | " | Betonschrauben | M 2ox8o | 1 | " |
| 37 | 1 | " | Antriebswelle | | 1 | " |
| 38 | 2 | " | Zahnriemen | | 1 | Kunstst. |
| 39 | 1 | " | Platte | | 1 | Metall |
| 4o | 12 | " | Senkschrauben | DIN 963-M 1ox25 | 1 | " |
| 41 | 4 | " | Sechskantschrauben | DIN 931-M 16x25o | 1 | " |
| 42 | 6 | " | Lager | | 1 | " |
| 43 | 2 | " | Achse | | 1 | " |
| 44 | 4 | " | Zahnriemenrad | | 1 | " |
| 45 | 3 | " | Abwasserbecken | | 1 | Blech |
| 46 | 4 | " | Stütze | | 1 | Metal |
| 47 | 1 | " | Motorstütze | | 1 | " |
| 48 | 1 | " | Trockner | Anfertigung | 1 | " |
| 49 | 1 | " | Zylinder | Anfertigung | 1 | " |

## Patentansprüche

1. Maschine zur Reinigung (B, C) bzw. Reinigung (B, C) und Desinfektion (D) von Bedienungsgeräten des Friseurgewerbes, wobei die Maschine wenigstens eine Kammer (B, C, D, E) aufweist und wobei die Maschine eine Abspritzvorrichtung zur Reinigung (b, C) bzw. Reinigung (B, C) und Desinfektion (D) sowie eine Trockenvorrichtung (E) aufweist, dadurch gekennzeichnet, daß die für einen Frisiervorgang benötigten Bediengeräte in einen oder mehrere Körbe (19) einbringbar sind und daß der Korb (19) bzw. die Körbe (19) in die Maschine einbringbar ist/sind (A) zur Reinigung (B, C) und gegebenenfalls Desinfektion (D) der in diesem Korb/diesen Körben (19) befindlichen Geräte, wobei die Maschine weiterhin eine Verschweißvorrichtung aufweist, mit der der Korb/die Körbe (19) in einem sich an die Trocknung (E) anschliessenden Arbeitsgang durch die Maschine durch Verschweißen mittels einer Folie verschliessbar ist/sind (G).

2. Maschine nach Anspruch 1,
dadurch gekennzeichnet, daß die Maschine mehrere Kammern (B, C, D, E) aufweist und daß verschiedene Kammern zum Abspritzen (B, C, D) und Trocknen (E) vorhanden sind.

3. Maschine nach einem der Ansprüche 1 oder 2,
dadurch gekennzeichnet, daß die Maschine mehrere Kammern aufweist und daß in der ersten als Spritzkammer ausgebildeten Kammer (B) die Geräte vorreinigbar sind, daß in der zweiten als Spritzkammer ausgebildeten Kammer (C) der Hauptspül- und Hauptwaschvorgang durchführbar ist unter Zugabe von Wärme und Reinigungsmittel, daß in der dritten als Spritzkammer ausgebildeten Kammer (D) die Bediengeräte überspülbar sind unter Beigabe von Desinfektionsmittel und daß in der vierten als Trockenkammer ausgebildeten Kammer (E) die Bediengeräte trockenbar sind unter Beimischung erwärmter Luft.

4. Verfahren zur Reinigung (B, C) bzw. Reinigung (B, C) und Desinfektion (D) von Bedienungsgeräten des Friseurgewerbes, mittels einer Maschine, die wenigstens eine Kammer (B, C, D, E) aufweist, wobei die Bedienungsgeräte zur Reinigung und Desinfektion abgespritzt (B, C, D) und getrocknet (E) werden,
dadurch gekennzeichnet, daß nach dem Einbringen der für einen Frisiervorgang benötigten Bediengeräte in einen oder mehrere Körbe (19) der Korb bzw. die Körbe (19) in die Maschine eingebracht werden (A) zur Reinigung (B, C) und gegebenenfalls Desinfektion (D) der in diesem Korb/diesen Körben (19) befindlichen Geräte, und wobei der Korb/die Körbe (19) nach der Trocknung (E) in der Maschine durch Verschweißen mittels einer Folie verschlossen wird/werden (G).

5. Verfahren nach Anspruch 4,
dadurch gekennzeichnet, daß die Maschine mehrere Kammern (B, C, D, E) aufweist und daß das Abspritzen (B, C, D) und das Trocknen (E) in verschiedenen Kammern erfolgt.

6. Verfahren nach Anspruch 4 oder 5,
dadurch gekennzeichnet, daß die Maschine mehrere Kammern (B, C, D, E) aufweist und daß in der ersten als Spritzkammer ausgebildeten Kammer (B) die Geräte vorgereinigt werden, daß in der zweiten als Spritzkammer ausgebildeten Kammer (C) der Hauptspül-und Hauptwaschvorgang durchgeführt wird unter Zugabe von Wärme und Reinigungsmittel, daß in der dritten als Spritzkammer (D) ausgebildeten Kammer die Bediengeräte überspült werden unter Beigabe von Desinfektionsmittel und daß in der vierten als Trockenkammer ausgebildeten Kammer (E) die Bediengeräte getrocknet werden unter Beimischung erwärmter Luft.

## Claims

1. A machine for the cleaning (B,C) or cleaning (B,C) and disinfecting (D) of hairdressing utensils, the machine having at least one chamber (B,C,D,E) and one spraying device for cleaning (B,C) or cleaning (B,C) and disinfecting (D) and also a drying device (E),
characterized in that the utensils required for a hairdressing operation can be put into one or more baskets (19) and that the basket/baskets (19) can be put into the machine (A) for the cleaning (B,C) and, as the case may be, disinfecting (D) of the utensils contained in this basket/these baskets (19), the machine also being equipped with a welding device with which, in an operation following on from the drying step (E), the basket/the baskets (19) can be sealed (G) by the machine with plastic sheeting.

2. The machine according to claim 1,
characterized in that the machine has a plurality of chambers (B,C,D,E) and that different chambers are provided for spraying (B,C,D) and drying (E).

3. The machine according to one of claims 1 or 2,
characterized in that the machine has a plurality of chambers and that in the first chamber (B), which is designed as a spraying chamber, the utensils can be pre-cleaned, that in the second chamber (C), also a spraying chamber, the main rinsing and washing operation can be carried out with addition of heat and cleansing agent, that in the third chamber (D), likewise a spraying chamber, the utensils can be rinsed with addition of disinfecting agent, and that in the fourth chamber, which is designed as a drying chamber (E), the utensils can be dried with warm air.

4. A process for the cleaning (B,C) or cleaning (B,C) and disinfecting (D) of hairdressing utensils by means of a machine which has at least one chamber (B,C,D,E), the utensils being cleaned and disinfected by spraying them down (B,C,D) and drying them (E),
characterized in that after the utensils required for a hairdressing operation have been put into one or more baskets (19), the basket or baskets (19) are put into the machine (A) for cleaning (B,C) and, as the case may be, disinfecting (D) of the utensils contained in this basket/these baskets (19), the basket/baskets (19) being sealed (G) by the machine with plastic sheeting, by means of welding, after the drying step (E).

5. The process according to claim 4,
characterized in that the machine has a plurality of chambers (B,C,D,E) and that the utensils are sprayed down (B,C,D) and dried (E) in different chambers.

6. The process according to claim 4 or 5,
characterized in that the machine has a plurality of chambers (B,C,D,E) and that in the first chamber (B), which is designed as a spraying chamber, the utensils are pre-cleaned, that in the second chamber (C), also a spraying chamber, the main rinsing and washing operation is carried out with addition of heat and cleansing agent, that in the third chamber (D), likewise a spraying chamber, the utensils are rinsed with addition of disinfecting agent, and that in the fourth chamber (E), which is designed as a drying chamber, the utensils are dried with warm air.

## Revendications

1. Machine pour nettoyer (B, C) ou nettoyer (B, C) et désinfecter (D) les instruments de coiffure, la machine présentant au moins une chambre (B, C, D, E) et la machine présentant un dispositif d'aspersion pour nettoyer (B, C) ou nettoyer (B, C) et désinfecter (D) ainsi qu'un dispositif de séchage (E), **caractérisé en ce que** les instruments nécessaires à une séance de coiffure peuvent être déposés dans un ou plusieurs paniers (19) et en ce que le panier (19) ou les paniers (19) peut / peuvent être introduits dans la machine (A) pour que les instruments placés dans le ou les panier (19) puissent être nettoyés (B, C) et le cas échéant désinfectés (D), la machine présentant de plus un dispositif de soudage avec lequel le panier / les paniers (19) peut / peuvent être scellé(s) (G) au moyen d'une pellicule par la machine lors d'une opération succédant au séchage (E).

2. Machine selon la revendication 1, **caractérisé en ce que** la machine présente plusieurs chambres (B, C, D, E) et qu'il est prévu différentes chambres pour asperger (B, C, D) et pour sécher (E).

3. Machine selon l'une des revendications 1 ou 2, **caractérisé en ce que** la machine présente plusieurs chambres et en ce que les instruments peuvent être prénettoyés dans la première chambre configurée comme chambre d'aspersion (B), en ce que le cycle principal de rinçage et de lavage peut être réalisé en ajoutant de la chaleur et du produit de lavage dans la seconde chambre configurée comme chambre d'aspersion (C), en ce que les instruments peuvent être rincés avec ajout de produit désinfectant dans la troisième chambre configurée comme chambre d'aspersion (D), et en ce que les instruments peuvent être séchés sous l'effet de l'air chaud dans la quatrième chambre configurée comme chambre de séchage (E).

4. Procédé pour laver (B, C) ou laver (B, C) et désinfecter (D) des instruments de coiffure au moyen d'une machine qui présente au moins une chambre (B, C, D, E), les instruments étant aspergés (B, C, D) pour le nettoyage et la désinfection et séchés, **caractérisé en ce que**, après que les instruments nécessaires à une séance de coiffure ont été placés dans un ou plusieurs paniers (19), le ou les paniers (19) sont introduits dans la machine (A) pour nettoyer (B, C) et le cas échéant désinfecter (D) les instruments se trouvant dans ce ou ces paniers (19), le ou les paniers étant fermés dans la machine à l'aide d'une pellicule par soudage (G) après le séchage (E).

5. Procédé selon la revendication 4, **caractérisé en ce que** la machine présente plusieurs chambres (B, C, D, E) et en ce que l'aspersion (B, D, D) et le séchage (E) se produisent dans des chambres différentes.

6. Procédé selon la revendication 4 ou 5, **caractérisé en ce que** la machine présente plusieurs chambres (B, C, D, E) et en ce que les instruments sont prénettoyés dans la première chambre configurée comme chambre d'aspersion (B), en ce que le cycle principal de rinçage et de lavage est réalisé en ajoutant de la chaleur et du produit de lavage dans la seconde chambre configurée comme chambre d'aspersion (C), en ce que les instruments sont rincés avec ajout de produit désinfectant dans la troisième chambre configurée comme chambre d'aspersion (D), et en ce que les outils sont séchés sous l'effet de l'air chaud dans la quatrième chambre configurée comme chambre de séchage (E).
